(19)

Europäisches
Patentamt
European
Patent Office
Office européen
des brevets

(11) **EP 4 563 724 A1**

(12) **EUROPEAN PATENT APPLICATION**
published in accordance with Art. 153(4) EPC

(43) Date of publication:
**04.06.2025 Bulletin 2025/23**

(21) Application number: **23845738.6**

(22) Date of filing: **31.07.2023**

(51) International Patent Classification (IPC):
***C23F 3/00*** (2006.01)

(52) Cooperative Patent Classification (CPC):
**C23F 3/00**

(86) International application number:
**PCT/CN2023/110247**

(87) International publication number:
**WO 2024/022534 (01.02.2024 Gazette 2024/05)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **29.07.2022 CN 202210907934**

(71) Applicant: **Biotyx Medical (Shenzhen) Co., Ltd.
Shenzhen, Guangdong 518000 (CN)**

(72) Inventors:
• LIU, Ziqiang
  Shenzhen, Guangdong 518000 (CN)
• ZHANG, Deyuan
  Shenzhen, Guangdong 518000 (CN)
• ZHANG, Gui
  Shenzhen, Guangdong 518000 (CN)

(74) Representative: **Prinz & Partner mbB
Patent- und Rechtsanwälte
Rundfunkplatz 2
80335 München (DE)**

(54) **POLISHING COMPOSITION AND USE THEREOF**

(57) Provided are a polishing composition and application thereof. Particularly, an efficient, stable, and high-precision polishing composition is related. The polishing composition includes an acid, an oxidizing agent, and water, where the acid is selected from inorganic acids, the oxidizing agent is selected from inorganic oxidizing agents, and a pH value of the polishing composition is ≤ 1. The polishing composition provided by the present invention is suitable for the polishing of medical devices, especially for the polishing of medical devices with a relatively large removal amount. The polishing composition is applied to polishing with the advantages of high polishing precision, simple and convenient operations, and low costs. Meanwhile, a polished product has good mechanical properties, surface properties, and biosafety.

**FIG. 2**

## Description

## Technical Field

The present invention relates to the field of pure chemical polishing, and particularly to an efficient, stable, and high-precision polishing composition.

## Background Art

In recent years, with the rapid development of the medical device industry, a large number of medical devices have been put into research and development. However, the majority of medical devices have high requirements for the smoothness of edges and the uniformity of the size and strength of various parts of the device during use. The polishing process is a key factor affecting the above-mentioned properties. Therefore, there are also high requirements for the polishing process and polishing composition of the medical device.

Currently, polishing methods commonly used for metal surface treatment include electrochemical polishing, chemical polishing, mechanical polishing, and chemical mechanical polishing. In the commonly used polishing methods mentioned above, in order to prevent a new polished surface formed after polishing from being quickly oxidized in the air and to ensure the stability of the surface of the device, the polishing process is usually divided into several steps, i.e., first polishing the polishing member, and then cleaning and passivating the new polished surface of the polishing member. The process is relatively complex. However, electrochemical polishing, due to the large difference in current densities and the like at different positions in the electrolytic cell, easily leads to a large difference in the polishing removal amounts at different positions of the pre-polishing member when the overall removal amount is large, which tends to result in low uniformity of the size of the pre-polishing member at various parts after polishing. Mechanical polishing or chemical mechanical polishing usually involves adding abrasives to the polishing composition and removing a part of the material of the pre-polishing member through mechanical grinding to achieve the corresponding effect. However, the abrasives tend to stick to and remain on the polishing member and leave scratches on the surface of the polishing member, resulting in the surface of the polishing member being not smooth enough. Therefore, mechanical polishing methods are not suitable for technical fields with strict product requirements, such as medical devices. Meanwhile, mechanical polishing methods have certain limitations on the size of the polishing member and are not suitable for the polishing of small-sized and complex-structured polishing members. Although chemical polishing has the advantages of low cost and simple operation, the commonly used inorganic acids, strong alkalis, and other components limit the application of chemical polishing.

Currently, in the field of medical devices, since the removal amount of the existing medical device is small in the polishing process, electrochemical polishing can also meet the requirements of the existing device in terms of uniformity, etc. Therefore, the commonly used method is still the electrochemical polishing method. For example, in patent CN102356184B, a method and solution for electropolishing stents made of high-strength medical alloys are disclosed. In this method, one or more conductive adapters need to be attached to each stent, and meanwhile, cathode and anode current conducting members are connected, which not only has high requirements for the apparatus, but also discloses in the examples thereof that the whole polishing process needs to be repeated four times. In addition, after polishing, a new polished surface formed after polishing is performed passivation treatment by immersing the rinsed stent into a passivation solution, thereby preventing the new polished surface from being rapidly oxidized in the air. The polishing process disclosed in the patent has problems such as complex processes and troublesome operations.

## Summary of the Invention

In order to overcome the above-mentioned drawbacks existing in the related art, the present invention provides a chemical polishing composition and application thereof. Components in the chemical polishing composition are relatively conventional compounds and have low costs. Meanwhile, polishing with this polishing composition not only simplifies the polishing process, making it easy to operate, but also provides mild polishing conditions, controllable and appropriate time, and good reproducibility, which is beneficial for the precision control of a polished stent. In addition, the polished polishing member has a uniform size at each position, a smooth surface, no burr, round edges, high precision, good stability, and good mechanical properties.

The technical solution of the present invention provides a polishing composition, including an acid, an oxidizing agent, and water. The acid is selected from at least one of inorganic acids. The oxidizing agent is selected from at least one of strong inorganic oxidizing agents. A pH value of the polishing composition is $\leq 1$. Further, the pH value of the polishing composition used in the present invention is not more than 0.8 nor more than 0.5. Further, the pH value of the polishing composition used in the present invention is not more than 0.2.

In the present invention, specific types of inorganic acids and strong oxidizing agents are adopted, and the contents thereof are controlled so that the oxidation and corrosion rates of the surface of a pre-polishing member may be sufficiently controlled, and meanwhile, the removal amount of the surface of the device may be increased to sufficiently remove cracks and the like generated inside the pre-polishing member due to the preceding process, thereby improving the mechanical properties of the polishing member to meet the requirements of various devices for the mechanical properties of the polishing member. In addition, the polishing efficiency of the polishing member may be sufficiently improved, and the polishing cost may be reduced.

The inorganic acid in the polishing composition provided by the present invention is selected from at least one of sulfuric acid, phosphoric acid, nitric acid, perchloric acid, or hydrofluoric acid. In some examples of the present invention, the inorganic acid in the polishing composition is sulfuric acid. In other examples of the present invention, the inorganic acid in the polishing composition is a mixed acid of phosphoric acid and nitric acid. In still other examples of the present invention, the inorganic acid in the polishing composition is perchloric acid.

The strong inorganic oxidizing agent in the polishing composition provided by the present invention is selected from at least one of sulfate, perchloric acid and salts thereof, perbromic acid and salts thereof, periodic acid and salts thereof, permanganic acid and salts thereof, peroxy acid salts, iodic acid and salts thereof, chlorous acid and salts thereof, hypochlorous acid and salts thereof, hypoiodous acid and salts thereof, oxybromic acid and salts thereof, percarbonate, bromic acid and salts thereof, chloric acid and salts thereof, hydrogen peroxide, potassium nitrate, or sodium nitrate. In some examples of the present invention, the strong inorganic oxidizing agent is one of the above-mentioned oxidizing agents. In other examples, the strong inorganic oxidizing agent is a mixture of two or more of the above-mentioned strong oxidizing agents.

In the above-mentioned technical solution provided by the present invention, a mass percentage content of the inorganic acid in the polishing composition is 40%-70%. Further, the mass percentage content of the inorganic acid in the polishing composition is 45%-70%, 48%-70%, 50%-70%, 53%-70%, 55%-70%, 58%-70%, 40%-65%, 40%-68%, 40%-63%, 40%-60%, 40%-58%, 40%-55%, 40%-53%, or 40%-50%. Further, the mass percentage content of the inorganic acid is 45%-65%, 45%-68%, 45%-63%, 45%-60%, 45%-58%, 45%-55%, 45%-53%, 45%-50%, 50%-65%, 55%-60%, or 50%-60%.

In the above-mentioned technical solution provided by the present invention, a mass percentage content of the oxidizing agent in the polishing composition is 5%-10%. Further, mass percentage content of the oxidizing agent in the polishing composition is 5%-8%, 5%-9%, 5%-9.5%, 5%-8.5%, 5.5%-9.5%, 5.5%-10%, 6%-10%, 6.5%-10%, 7%-10%, 5%-7%, 6%-9%, or 6%-8%. Further, the mass percentage content of the oxidizing agent in the polishing composition is 6%-7% or 5%-6%.

In the present invention, the roundness of the polished device is controlled by controlling the viscosity of the polishing solution. In the present invention, the viscosity of the polishing solution is controlled within a certain range so that the sharp edge of the device may be sufficiently polished to be smooth and round without burr to ensure that the medical device meets the corresponding safety requirements, and pits and the like formed on the surface of the device due to excessive polishing and removal at a certain position of the device is prevented, thereby ensuring the smoothness and flatness of the surface of the device. Meanwhile, the case that the polishing member cannot in the polishing solution due to too viscous polishing solution is prevented.

The technical solution of the present invention puts forward a high requirement for the viscosity of the polishing solution. The viscosity of the polishing solution cannot be too high. If the viscosity of the polishing solution is too high, the rotation of the stent will be affected, thereby affecting the polishing speed and polishing uniformity of the stent. Meanwhile, too viscous polishing solution cannot flow smoothly on the inner wall of the stent so that the stent has problems such as insufficient internal polishing, deep scratches on the inner wall, poor appearance of polishing, and poor mechanical properties of the polished stent. However, if the viscosity of the polishing solution is insufficient, the polished stent is not round, and the removal effect of the burr on the surface of the stent and the scratches inside the matrix of the stent is poor, etc.

In the above-mentioned technical solution provided by the present invention, the viscosity of the polishing solution is 1.1 mPa s-4 mPa s. Further, the viscosity of the polishing solution is 1.5 mPa s-4 mPa s, 1.2 mPa s-4 mPa s, 1.4 mPa s-4 mPa s, 1.6 mPa s-4 mPa s, 1.2 mPa s-3.5 mPa s, 1.4 mPa s-3.5 mPa s, 1.6 mPa s-3.5 mPa s, 1 mPa s-3.5 mPa s, 1.8 mPa s-3.5 mPa s, 2.0 mPa s-3.5mPa s, 1.2 mPa s-3.4 mPa s, 1.2 mPa s-3.4 mPa s, 1.2 mPa s-3.2 mPa s, 1.2 mPa s-3.6 mPa s, 1.2 mPa s-3.0 mPa s, and 1.4 mPa s-3.3 mPa s. Further, the viscosity of the polishing solution is 1.4 mPa s-3.0 mPa s, 1.4 mPa s-3.2 mPa s, 1.6 mPa s-3.0 mPa s, and 1.6 mPa s-2.9 mPa s. In some examples of the present invention, the viscosity of the polishing solution is 1.9 mPa s, 1.7 mPa s, 2.1 mPa s, 2.3 mPa s, 2.5 mPa s, 2.7 mPa s, 2.9 mPa s, 3.0 mPa s, 3.1 mPa s, or 3.3 mPa s. In other examples, the viscosity of the polishing solution is 3.5 mPa s, 3.7 mPa s, or 3.9 mPa s.

In the above-mentioned technical solution of the present invention, the viscosity of the polishing solution is further controlled using a viscosity agent. A mass percentage content of the viscosity agent is 1.5%-7%. Further, the mass percentage content of the viscosity agent is 2%-7%, 2.5%-7%, 3%-7%, 3.5%-7%, 4.5%-7%, 4%-7%, 3.5%-6.5%, 2.5%-6.5%, 2.5%-7%, 2%-6.5%. Further, the mass percentage content of the viscosity agent is 2%-6%, 2.5%-6%, 3%-6%, 4%-6%, 3.5%-6%, and 1.5%-6%.

In the above-mentioned technical solution provided by the present invention, the viscosity agent in the polishing composition is selected from at least one of alcohols, silicic acid, silicate, alginic acid, alginate, polyvinylpyrrolidone, polyvinylpyrrolidone-modified organic, ethyl cellulose, methyl cellulose, hydroxyethyl cellulose, hydroxypropylmethyl cellulose, carboxymethyl cellulose, polyacrylic acid, sodium polymethacrylate, polycarboxylic acid sodium salts, carboxylic acid sodium salt copolymers, carboxylic acid sulfonic acid copolymers, guar gum, sodium starch phosphate, sodium polyacrylate, polyoxyethylenes, carbomer, xanthic acid, arabic gum, carrageenan, agar, gelatin, acacia gum, and derivatives thereof. For example, in some embodiments of the present invention, the viscosity agent in the polishing composition is silicic acid. In other examples, the viscosity agent is hydroxypropylmethyl cellulose. In still other examples, the viscosity agent is a combination of an acacia gum derivative and an alcohol.

In the above-mentioned technical solution of the present invention, the viscosity agent in the polishing composition includes at least one of A components and at least one of B components, wherein

the A component is selected from at least one of alcohols, ethyl cellulose, methyl cellulose, hydroxyethyl cellulose, hydroxypropylmethyl cellulose, carboxymethyl cellulose, polyvinylpyrrolidone, polyacrylic acid, polymethacrylate, polycarboxylic acid sodium salts, carboxylic acid sodium salt copolymers, carboxylic acid sulfonic acid copolymers, and derivatives thereof;

the B component is selected from a mixture of at least one of silicic acid, silicate, alginic acid, alginate, polyvinylpyrrolidone-modified organic, guar gum, sodium starch phosphate, sodium polyacrylate, polyoxyethylenes, carbomer, xanthic acid, arabic gum, carrageenan, agar, gelatin, acacia gum, and derivatives thereof.

In some embodiments of the present invention, the viscosity agent is ethyl cellulose and guar gum. In other examples, the viscosity agent is sodium silicate and sodium polymethacrylate.

In the above-mentioned technical solution provided by the present invention, a mass ratio of the A component to the B component of the viscosity agent in the polishing composition is 0.5: 1-15: 1. Further, the mass ratio of the A component to the B component of the viscosity agent is 0.5: 1-10: 1. Further, the mass ratio of the A component to the B component of the viscosity agent is 1.5: 1-10: 1.

In the above-mentioned technical solution provided by the present invention, the molecular weight Mn of the alcohol in the viscosity agent is $\leq$ 800 g/mol. Further, the molecular weight of the viscosity agent is $\leq$ 720 g/mol. Further, the alcohols and derivatives thereof in the present invention refer to alcohols which are liquid at normal temperature, and may be straight-chain alcohols or branched-chain alcohols. The position of the hydroxyl group is also not fixed, such as butanediol, which may be arranged in a straight chain of four methyl groups, methylene groups and/or methylidyne groups, or the carbon chain contains quaternary carbon atoms. In addition, the positions of the two hydroxyl groups may also be arbitrarily transformed on the four carbon atoms, and so on.

In the above-mentioned technical solution provided by the present invention, the alcohols and derivatives thereof are at least one of a monohydric alcohol monomer and a polyhydric alcohol monomer having no more than 6 carbon atoms, and a polymer formed by the monohydric alcohol monomer and the polyhydric alcohol monomer having no more than 6 carbon atoms.

It should be noted that no more than or not more than in the present invention means either less than or equal to, and "monohydric alcohol and polyhydric alcohol having no more than 6 carbon atoms" described in the present invention refers to a monohydric alcohol or polyhydric alcohol having less than or equal to 6 carbon atoms.

In the above-mentioned technical solution provided by the present invention, the alcohols and derivatives thereof are at least one of ethylene glycol, propylene glycol, glycerol, butanediol, polyethylene glycol, butanol, cyclohexanol, polypropylene glycol, and polybutylene glycol.

In the above-mentioned technical solution provided by the present invention, a specific type and mass content of complexing agent may be further added to the polishing composition containing an inorganic acid and a strong oxidizing agent so that the complexing agent may be sufficiently combined with metal ions polished off to avoid an excessively high metal ion content, thereby sufficiently prolonging the service life of the polishing solution.

In the above-mentioned technical solution provided by the present invention, a mass percentage content of the complexing agent is 0.5%-2%. Further, the mass percentage content of the complexing agent is 1%-2%.

In the above-mentioned technical solution provided by the present invention, the complexing agent has a monodentate or polydentate ligand. The complexing agent is selected from at least one of an amino group, a carboxyl group, a cyanide group, a thiocyanate group, an isothiocyanate group, a nitro group, a hydroxyl group, a mercapto group, an aromatic heterocyclic group, a nitroso group, a sulfo group, a phosphoric acid group, and an organic phosphine group.

In the above-mentioned technical solution provided by the present invention, the complexing agent is selected from at least one of hydroxycarboxylic acid, aminocarboxylic acid, hydroxyaminocarboxylic acid, hydroxyphosphonic acid, dicarboxylic acid, and salts thereof.

In the above-mentioned technical solution provided by the present invention, the complexing agent is selected from at least one of oxalic acid, amino trimethylene phosphonic acid, hydroxyethylidene diphosphonic acid, ethylenediamine tetramethylene phosphonic acid, amino trimethylene phosphonic acid, diethylene triamine pentacarboxylic acid, ethylenediamine tetramethylene phosphonic acid, ethylenediamine tetraacetic acid, diethylene triamine pentamethylene phosphonic acid, amino trimethylene phosphonic acid, hydroxyethylidene diphosphonic acid, 2-hydroxy phosphonoacetic acid, dihexene triamine pentamethylene phosphonic acid, 2-phosphono-1,2,4-butane tricarboxylic acid, heptanoic acid, 2-phosphonobutane-1,2,4-tricarboxylic acid, itaconic acid, succinic acid, tartaric acid, maleic acid, glycolic acid, malonic acid, oxalic acid, malic acid, gluconic acid, alanine, glycine, lactic acid, diethylenetriamine pentaacetic acid, triethylenediamine, propylenediamine tetraacetic acid, hydroxyethyl ethylenediamine, hydroxyethyl ethylenediamine triacetic acid, pyrophosphoric acid, 2-aminoethylphosphonic acid, 1-hydroxyethylidene 1,1-diphosphonic acid, amino trimethylene phosphonic acid, ethylenediamine tetramethylene phosphonic acid, diethylenetriamine pentamethylene phosphonic acid, ethane -1,1-diphosphonic acid, ethane -1,1,2-triphosphonic acid, methane hydroxyphosphonic acid, 1-phosphonobutane-2,3,4-tricarboxylic acid and salts thereof, dihydroxy glycine, disodium EDTA, succinic acid, polyvinyl alcohol, triethanolamine, and salts thereof.

It should be noted that a component of the polishing composition of the present invention is selected from at least one of a, b, c, d, and derivatives thereof, or a component of the polishing composition of the present invention is selected from at least one of a, b, c, d, and salts thereof, which means that a component of the polishing composition is selected from at least one of a, a derivative, b, b derivative, c, c derivative, d, and d derivative, or a component of the polishing composition is selected from at least one of a, a salt, b, b salt, c, c salt, d, and d salt. For example, "the complexing agent is selected from at least one of hydroxycarboxylic acid, aminocarboxylic acid, hydroxyaminocarboxylic acid, hydroxyphosphonic acid, dicarboxylic acid, and salts thereof" refers to that the complexing agent is selected from at least one of hydroxycarboxylic acid, aminocarboxylic acid, hydroxyaminocarboxylic acid, hydroxyphosphonic acid, dicarboxylic acid, a hydroxycarboxylic acid salt, an aminocarboxylic acid salt, a hydroxyaminocarboxylic acid salt, a hydroxyphosphonic acid salt, and a dicarboxylic acid salt.

Salts described in the present invention refers to salts formed by the corresponding acids with respective metal ions, including sodium salts, potassium salts, ammonium salts, calcium salts, and magnesium salts. For example, the alginate includes sodium alginate, potassium alginate, ammonium alginate, calcium alginate, and magnesium alginate. The silicate includes aluminum silicate, iron silicate, calcium silicate, magnesium silicate, potassium silicate, sodium silicate, and so on.

In the technical solution provided by the present invention, a surface protecting agent may be further added to the polishing composition containing an inorganic acid and an oxidizing agent to ensure that a thin protective film may be formed on the surface of the polishing member after polishing, thereby preventing the polishing member from being oxidized in the air and ensuring that the polishing member remains bright and smooth for a long period of time. Meanwhile, the chemical stability of the polishing member may be improved, thereby prolonging its shelf life or service life.

In the above-mentioned technical solution provided by the present invention, the polishing composition further includes a surface protecting agent. A mass percentage content of the surface protection agent is 0.02%-1%. Further, the mass percentage content of the surface protection agent is 0.05%-0.7%, 0.05%-0.8%, 0.05%-0.6%, 0.05%-0.5%, 0.05%-0.4%, 0.05%-0.3%, 0.05%-0.2%, 0.05%-0.1%, 0.1%-0.8%, 0.1%-0.7%, 0.1%-0.6%, 0.1%-0.5%, 0.1%-0.4%, 0.1%-0.3%, 0.1%-0.9%, or 0.05%-1%.

In the above-mentioned technical solution provided by the present invention, the surface protecting agent is selected from at least one of tannic acid, phytic acid, stearic acid, palmitic acid, and salts thereof. That is, the surface protecting agent may be at least one of tannic acid, phytic acid, stearic acid, palmitic acid, tannate, phytate, stearate, and palmitate, or may be a mixture of at least one of tannic acid, phytic acid, stearic acid, and palmitic acid and at least one of tannate, phytate, stearate, and palmitate. Salts include, but are not limited to, sodium salts, potassium salts, ammonium salts, calcium salts, or magnesium salts.

In the technical solution provided by the present invention, a specific type and mass content of surfactant may be further added to the polishing composition containing an inorganic acid and a strong oxidizing agent so that the surface tension of the solution may be reduced through the surfactant. Thus, the components in the polishing solution are sufficiently dispersed, thereby facilitating the flow of the polishing solution on the inner wall and the outer wall of the stent. Therefore, the ingredients of the polishing solution near various parts of the pre-polishing member are substantially the same, thereby ensuring that the effects of the polishing solution on various parts of the pre-polishing member are substantially uniform, and finally ensuring the uniformity of the surface of the finished polishing member. In addition, the surfactant also has a certain thickening effect on water, and meanwhile, it has a great affinity and wettability to the surface of the polishing member. The surfactant can not only penetrate into the metal surface to increase the polishing effect of the polishing solution on the metal, but also repair the pits, damages, and uneven parts on the surface of the polishing member, thereby improving the flatness and brightness of the workpiece, and making the surface of the polishing member bright and uniform.

In the above-mentioned technical solution provided by the present invention, the surfactant is at least one of an anionic surfactant, a cationic surfactant, a nonionic surfactant, and an amphoteric surfactant.

In the above-mentioned technical solution provided by the present invention, a mass percentage content of the surfactant is 0.05%-1%. Further, the mass percentage content of the surfactant is 0.05%-0.7%, 0.05%-0.8%, 0.05%-0.6%, 0.05%-0.5%, 0.05%-0.4%, 0.05%-0.3%, 0.05%-0.2%, 0.05%-0.1%, 0.1%-0.8%, 0.1%-0.7%, 0.1%-0.6%, 0.1%-0.5%. 0.1%-0.4%, 0.1%-0.3%, 0.1%-0.9%, or 0.05%-1%.

In the above-mentioned technical solution provided by the present invention, the surfactant is selected from at least one of higher fatty acid salts, alkyl sulfonate, alkyl ether carboxylate surfactants, silicone surfactants, alkane sulfate, alkane sulfonate, substituted amine salts, betaine, polyethylene oxide, polyvinyl alcohol, polyvinyl acetate, polyacrylic acid, polyvinyl pyrrolidone, lecithin, amino acid derivatives, alkylphenol polyoxyethylene ether, fatty alcohol polyoxyethylene ether sodium sulfate, isooctanol polyoxyethylene ether, alkyl glucoside, polyoxyethylene ether, polyethyleneimine, alkyl trimethyl quaternary ammonium salts, alkyl dimethyl benzyl ammonium chloride salts, or pyridinium salts.

In the above-mentioned technical solution provided by the present invention, the surfactant is selected from at least one of sodium dodecyl sulfate, sodium dodecyl sulfonate, OP-10, linear alkylbenzene sulfonate (LAS), fatty alcohol polyoxyethylene ether sulfate (AES), fatty alcohol polyoxyethylene ether ammonium sulfate (AESA), sodium lauryl sulfate (SDS), lauroyl glutamic acid, nonylphenol polyoxyethylene ether (TX-10), glycerol monostearate, lignosulfonate, heavy alkylbenzene sulfonate, alkyl sulfonate, diffusant NNO, diffusant MF, alkyl polyether (PO-EO copolymer), fatty alcohol polyoxyethylene ether (AEO-3), betaine, alkyl trimethyl quaternary ammonium salts, lecithin, amino acid derivatives, alkylphenol polyoxyethylene ether, fatty alcohol polyoxyethylene ether sulfate, isooctanol polyoxyethylene ether, alkyl glucoside, polyoxyethylene ether, alkyl dimethyl benzyl ammonium chloride salts, or pyridinium salts.

In the above-mentioned technical solution provided by the present invention, the present invention further provides an application of the polishing composition in polishing a medical device.

In the above-mentioned technical solution provided by the present invention, the mass of the medical device is relatively small, and the mass of a single medical device polishing member is generally less than 0.5 Kg. The single medical device polishing member herein may be a part of a particular medical device or may be an entire medical device.

In the above-mentioned technical solution provided by the present invention, the medical device is a degradable medical device.

In the above-mentioned technical solution provided by the present invention, the medical device includes an iron-containing medical device. The iron-containing medical device may refer to a device entirely composed of iron elements or a device composed of iron elements and other elements. That is, the iron-containing medical device may be made of a pure iron material or an iron-containing alloy. The iron-containing medical device may be that the whole device is made of an iron-containing material, or a certain component or a certain part of the device is made of an iron-containing material.

In the above-mentioned technical solution provided by the present invention, the medical device includes an interventional material/device or an implant material/device used in vivo.

In the above-mentioned technical solution provided by the present invention, the medical device is made of a degradable metal or a metal alloy. The medical device includes a vascular stent, a non-vascular endoluminal stent, an occluder, a spacer, an artificial vessel, a dental implant device, a vascular clamp, an artificial heart valve, an orthopedic implant, a dental implant, a respiratory implant, a gynecological implant, an andrological implant, a suture, or a bolt. The orthopedic implant includes a bone nail, a bone plate, and the like.

The polishing composition provided by the present invention is suitable for the polishing of metals, particularly for the polishing of iron bases or iron-based alloys. The iron-based alloys include low alloy steels or iron-based alloys with a carbon content of not more than 2.5 wt.%. Further, the polishing composition provided by the present invention is suitable for the polishing of relatively small-sized and complex-structured products.

In the above-mentioned technical solution provided by the present invention, a mass-to-volume ratio of the medical device polishing member is 0.001 $g/cm^3$-0.4 $g/cm^3$. Further, the mass-to-volume ratio of the medical device polishing member is 0.002 $g/cm^3$-0.3 $g/cm^3$. Further, the mass-to-volume ratio of the medical device polishing member is 0.005 $g/cm^3$-0.25 $g/cm^3$.

In the above-mentioned technical solution provided by the present invention, the iron-containing medical device is made of pure iron or an iron-based alloy with a carbon content of not more than 2.11 wt%.

In the present invention, "/" means "or". For example, "interventional material/device" means "interventional material or interventional device".

It should be noted that the mass percentage content of each component described in the present invention refers to the mass percentage of a core ingredient of each component as a solute throughout the polishing composition. For example, if a component M itself is a solution and its solute is J, the mass percentage content of the component in the polishing composition refers to the mass percentage of J throughout the polishing composition K, rather than the mass percentage of M throughout the polishing composition. That is, the mass percentage content is the percentage of the mass of J to the mass of the composition K.

It should be noted that the mass percentages of the components in the polishing composition of the present invention plus the mass percentages of water is 100%, i.e., the composition is a mixed solution including the core components and water.

In the present invention, the components are rationally matched, and the relative contents of the components are controlled so that the components work together and cooperate with each other to finally ensure that the polishing member has a smooth, round, and bright surface without burrs and edges, thereby reducing the irritation and damage of the device to the human body. Meanwhile, the mechanical properties of the device may be sufficiently improved to ensure that the device can meet the corresponding mechanical property requirements in various application scenarios.

The polishing composition provided in the present invention not only has a high polishing efficiency but also ensures that various parameters of each two or more polishing members remain stable after polishing. The polishing precision is high to ensure a controllable product weight.

The polishing composition provided in the present invention polishes the polishing member under a certain condition so that the removal amount of the polishing member after polishing may reach 20%-60%, and meanwhile, the polishing uniformity of each part of the device may reach 85%, 90%, 95%, or even more than 98%. Each part of the polished polishing member has a bright and smooth surface without scratches at a magnification of 200 times under an optical microscope and shows no signs of fractures after the polishing member is bent several hundred times, pulled up, and expanded several times.

Components in the polishing composition provided by the present invention are relatively conventional compounds and have low costs. Polishing with this polishing composition not only simplifies the polishing process, making it easy to operate, but also provides mild polishing conditions, controllable and appropriate time, and good reproducibility, which is beneficial for the precision control of the polished stent. A plurality of identical stents are polished under the same conditions, and the RSD of the masses of the plurality of polished stents is within 2%, even within 0.8%.

According to the polishing composition provided by the present invention, the polished polishing member has a uniform size at each position, a smooth surface, no burr, round edges, high precision, good stability, and good mechanical properties.

The polishing process of the present invention is simple in operation, which only requires placing the pre-polishing member into a prepared polishing solution and polishing for several minutes or tens of minutes under certain conditions, without subsequent passivation and other steps, so as to obtain a high-quality polishing member. The polishing member has high polishing stability and repeatability.

According to the polishing composition provided by the present invention, a finished polishing member polished in the polishing composition has a slight scratch on a surface after being magnified 500 times under a scanning electron microscope (SEM), where a ratio of a scratch area to a surface area of the whole polishing member is ≤ 20%. Further, according to the polishing member provided by the present invention, the scratch area on the surface of the polishing member is not more than 15%, 10%, or even 6% of the surface area of the whole polishing member at a magnification of 500 times under the SEM.

According to the polishing composition provided by the present invention, a scratch depth h of the finished polishing member polished in the polishing composition is ≤ 2.5 $\mu$m. Further, the scratch depth of the polishing member may be less than 1 $\mu$m. Further, the scratch depth of the polishing member may be less than 0.5 $\mu$m or less than 0.3 $\mu$m.

According to the polishing composition provided by the present invention, the roughness Sa of the surface of the finished polishing member polished in the polishing composition is ≤ 50 nm. Further, the roughness Sa of the surface of the medical device polishing member is not more than 30 nm. Further, the roughness Sa of the surface of the medical device polishing member is not more than 20 nm.

According to the polishing composition provided by the present invention, a thin protective film is attached to the surface of the finished polishing member polished in the polishing composition so that the surface of the polishing member has relatively good stability, is not easily oxidized by air, etc., thereby ensuring the stability of the surface appearance and property of the device polishing member during transit and storage.

According to the polishing composition provided by the present invention, the finished polishing member polished in the polishing composition has a smooth and bright surface after being magnified 100 times under an optical electron microscope. Further, the polishing member still has a smooth and bright surface after being magnified 200 times or 250 times under the optical electron microscope.

The polishing composition provided by the present invention may sufficiently control the polishing removal amount of various parts of the medical device polishing member in the polishing process to ensure that the polishing uniformity of the polishing member may reach 85% or more. Preferably, the polishing uniformity of the polishing member is controlled to 90%, 95%, even 98% or more, thereby ensuring that the whole medical device polishing member has no weak parts and has good mechanical properties. In the present invention, the polishing uniformity of the polishing member refers to the relatively uniform size of the polished polishing member. That is, the polishing uniformity refers to a ratio of the polished width/thickness $R_i$ of the polishing member at the same part after polishing to an average value of the polished width/thickness at the same part after polishing,

i.e., $\dfrac{R_i}{R_{\text{average}}}$. For example, the uniformity of the polished size of the medical device polishing member at each part in the polishing process is more than 85%,

$$\frac{R_i}{R_{\text{average}}} \geq 85\%$$

i.e., .

The polishing composition provided by the present invention has high stability in the polishing process of the polishing member. After several tens of stents are polished simultaneously, the stents still have good appearances, and the size, weight, and other parameters of the stents are still stable.

It should be noted that, for the sake of clarity, the advantages of the technical solution of the present invention are described separately. However, the solution provided by the present invention is an indivisible whole. The desired final effect can only be achieved through the mutual coordination and interaction of the components. Without the support of other components, a single component cannot independently achieve a very good effect. For example, although the viscosity agent in the present invention may improve the smoothness of the surface of the polishing member, the viscosity of the solution is the result of the interaction of all components, not the result of the action of only the viscosity agent. That is, the smoothness of the surface of the polishing member after the final polishing is inseparable from the interaction of the components.

In the present invention, the value of the interval range is not limited to the interval range provided, but should be the value of a new interval composed of any two values in the interval or any specific value in the interval. For example, "the content of the surfactant is 0.05%-0.1%" in the present invention refers to that the value of the content of the surfactant is not limited to the interval range of 0.05-1% and may be a new interval composed of any two values in the infinite number of values between 0.05%-1%, such as may be 0.05%-0.5% or 0.1%-0.8%. In addition, when there are a plurality of numerical combinations, each parameter may have any value within its value range, and the values of the plurality of parameters may be arbitrarily matched. For example, the pH value of the polishing solution is $\leq 1$, and the viscosity of the polishing solution is 1-4 mPa s. The pH value of the polishing solution may be any value within a range of values not greater than 1, and the viscosity of the polishing solution may have any value within a range of 1-4 mPa s. The two parameters may also be arbitrarily matched. For example, the pH value is 0.1, and the viscosity of the polishing solution is 3.5 mPa s, and so on.

It should be understood that the terms used herein are for the purpose of describing particular exemplary embodiments only and are not intended to form a limitation. As used herein, the singular forms "a", "an", and "the" are intended to include the plural forms as well, unless the context clearly indicates otherwise. The terms "comprising", "including", "containing", and "having" are inclusive, and therefore specify the presence of stated features, steps, operations, elements, and/or components, but do not preclude the presence or addition of one or more other features, steps, operations, elements, components, and/or combinations thereof. The method steps, processes, and operations described herein are not to be construed as necessarily requiring that they be performed in the particular order described or illustrated, unless the order of performance is explicitly stated. It should also be understood that additional or alternative steps may be used.

In the description of this specification, the description with reference to the terms "one example", "some examples", "implementation", "particular implementations", or "some implementations" means that specific features, structures, materials, or characteristics described in connection with the example or implementation are included in at least one example or implementation of the present application. In this specification, schematic expressions of the above-mentioned terms do not necessarily refer to the same example or implementation. Furthermore, the specific features, structures, materials, or characteristics described may be combined in any suitable manner in any one or more examples or implementations. In addition, the different examples or implementations and the features of the different examples or implementations described in this specification may be combined by a person skilled in the art without contradicting each other.

**Brief Description of the Drawings**

Various other advantages and benefits will become apparent to a person skilled in the art upon reading the following detailed description of the preferred embodiments. The drawings are only for the purpose of illustrating the preferred embodiments and are not to be construed as limiting the present invention. Moreover, throughout the accompanying drawings, the same components are indicated by the same reference numerals.

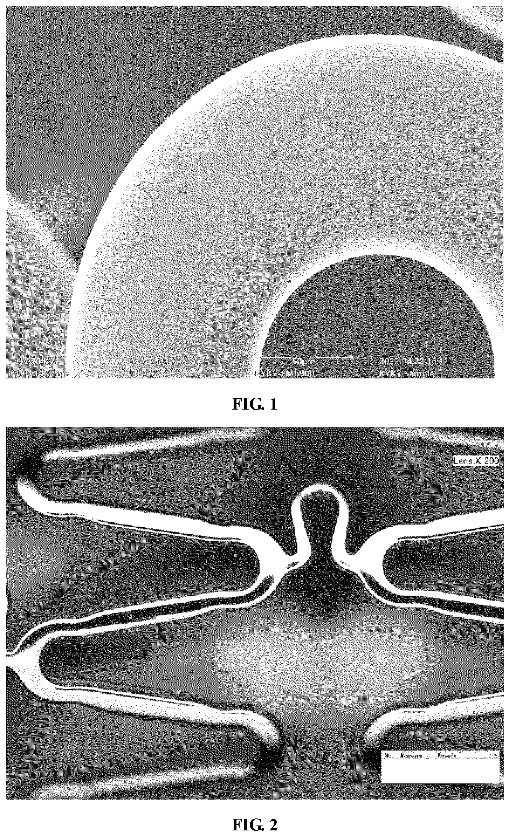

FIG 1 is a characterization diagram of a stent of example 1 at a magnification of 500 times under an SEM;

FIG 2 is a characterization diagram of a stent of example 1 at a magnification of 200 times under an optical microscope; and

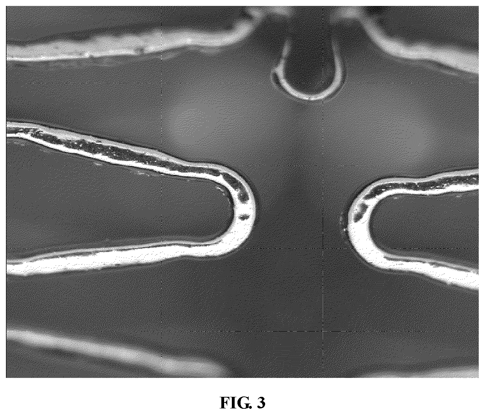

FIG 3 is a characterization diagram of a stent of comparative example 1 at a magnification of 200 times under an optical microscope.

**Detailed Description of the Invention**

The following descriptions are only preferred embodiments of the present invention, and the protection of the present invention is not limited to the following preferred embodiments. For example, in the examples, the description is given by taking a stent or a stent of a certain model as an example, but it does not represent that the technical solution of the present invention is only suitable for the stent or the stent of a certain model. It should be noted that several variations and improvements made by a person skilled in the art based on the present inventive concept fall within the scope of the present invention. The reagents or instruments used are conventional products that are commercially available through regular channels without specifying the manufacturer.

**Test methods**

1. Roughness

In the present invention, the roughness of a surface of a polishing member is measured using a Q-SIX cardiovascular stent detector manufactured by SENSOFAR under an interference lens at a magnification of 400 times and a scanning height of 50 $\mu$m.

2. Viscosity

A polishing solution is poured into a Zahn cup CUP1#, and the outflow time t is recorded. The viscosity of the solution is calculated according to the formula: dynamic viscosity = 1.1(t-29) * $\rho$ ($\rho$ refers to the solution density).

3. Measurement of width and thickness of stent

A stent is placed under an optical microscope, and the size of the stent is observed and measured under the optical microscope at a magnification of 100-200 times.

4. Scratch depth

The stent is sealed and then polished using a polishing machine until a cross section of the stent is exposed. Finally, the polished cross section is placed under a metallographic microscope to measure the scratch depth.

5. Radial strength

In the property test of the lumen stent, the radial strength of the lumen stent can be tested by applying radial pressure uniformly to the stent using a compression module, causing the stent to compress and undergo uniform deformation. The radial strength of the stent is defined as the magnitude of the radial pressure applied when 10% deformation occurs in the radial direction (outer diameter) of the stent.

6. Over-expansion plasticity

A balloon catheter with the corresponding length and suitable outer diameter is selected for the stent. The balloon catheter is pressurized and expanded from 6-8 atm, and after holding the pressure for 30 s, the whole stent is traversed under a 200$\times$ three-dimensional measuring microscope to record whether there are cracks/broken rods. If there are no cracks/broken rods, the operation of holding the pressure for 30 s after pressurizing for 2 atm for observation and recording is repeated until cracks/broken rods are found or the expanded outer diameter of the stent reaches the acceptance criteria.

7. Appearance

The brightness and roughness of the outer and inner walls of the whole stent are fully examined through the three-dimensional measuring microscope at an appropriate magnification (such as 50-200 times).

**Example 1**

A 30018 stent was cut, placed in an ultrasonic wave for cleaning for 2 min, and then placed in a polishing solution with a viscosity of 4.0 mPa s, which was prepared by mixing 100 ml of sulfuric acid, 3 g of EDTA, 10 ml of glycerol, 5 g of sodium nitrate, 5 g of sodium silicate, 0.1 ml of phytic acid, 0.05 g of sodium dodecyl sulfate, and 50 ml of water. A flow rate of the polishing solution was controlled to be 1.0 m/s, and the stent was polished at 20°C for 3 min. The stent was removed, cleaned, and dried. The dried stent was observed under an optical microscope with a magnification of 200 times to find that the surface was smooth and bright. The dried stent was observed under an SEM with a magnification of 500 times to find that edges of the stent were round, the percentage of a surface scratch area was 2%, the maximum depth of the scratch was 1 $\mu$m, the single-side removal depth of a stent rod was 15 $\mu$m, and the uniformity of stent polishing was 90%. A mass loss rate of the stent was 45%, and the roughness Sa of the surface of the stent measured under a sensofar 3D optical profiler was 15 nm. The stent had a radial strength of 120 kPa and was expanded using a balloon to a diameter of 4.6 mm without cracks and fracture. Five stents were consecutively and repeatedly polished, and the RSD of the mass of the polished stents was < 1%.

**Example 2**

A 30018 stent was cut, placed in an ultrasonic wave for cleaning for 2 min, and then placed in a polishing solution with a viscosity of 2.0 mPa s, which was prepared by mixing 100 ml of hydrochloric acid, 5 g of tartaric acid, 10 ml of ethylene glycol, 10 ml of perchloric acid, 5 g of sodium alginate, 0.1 g of sodium phytate, 0.05 g of OP-10, and 50 ml of water. A flow rate of the polishing solution was controlled to be 0.8 m/s, and the stent was polished at 25°C for 3 min. The stent was removed, cleaned, and dried. The dried stent was observed under an optical microscope with a magnification of 200 times to find that the surface was smooth and bright. The dried stent was observed under an SEM with a magnification of 500 times to find that edges of the stent were round, the percentage of a surface scratch area was 2%, the maximum depth of the scratch was 0.5 $\mu$m, the single-side removal depth of a stent rod was 15 $\mu$m, and the uniformity of stent polishing was 95%. A mass loss rate of the stent was 42%, and the roughness Sa of the surface of the stent measured under a sensofar 3D optical profiler was 10 nm. The stent had a radial strength of 115 kPa and was expanded using a balloon to a diameter of 4.6 mm without cracks and fracture. Five stents were consecutively and repeatedly polished, and the RSD of the mass of the polished stents was < 1.5%. Five stents were consecutively and repeatedly polished, and the RSD of the mass of the polished stents was < 1.5%.

## Example 3

A 30018 stent was cut, placed in an ultrasonic wave for cleaning for 5 min, and then placed in a polishing solution with a viscosity of 3.0 mPa s, which was prepared by mixing 100 ml of phosphoric acid, 5 g of disodium EDTA, 10 ml of butanediol, 10 ml of nitric acid, 5 g of sodium silicate, 0.1 ml of tannic acid, 0.05 g of polyethylene glycol, and 50 ml of water. A flow rate of the polishing solution was controlled to be 0.5 m/s, and the stent was polished at 30°C for 3 min. The stent was removed, cleaned, and dried. The dried stent was observed under an optical microscope with a magnification of 200 times to find that the surface was smooth and bright. The dried stent was observed under an SEM with a magnification of 500 times to find that edges of the stent were round, the percentage of a surface scratch area was 2%, the maximum depth of the scratch was 1.5 $\mu$m, the single-side removal depth of a stent rod was 15 $\mu$m, and the uniformity of stent polishing was 92%. A mass loss rate of the stent was 45%, and the roughness Sa of the surface of the stent measured under a sensofar 3D optical profiler was 20 nm. The stent had a radial strength of 125 kPa and was expanded using a balloon to a diameter of 4.6 mm without cracks and fracture. Five stents were consecutively and repeatedly polished, and the RSD of the mass of the polished stents was < 2%.

## Example 4

A 30018 stent was cut, placed in an ultrasonic wave for cleaning for 2 min, and then placed in a polishing solution with a viscosity of 1.5 mPa s, which was prepared by mixing 100 ml of hydrochloric acid, 3 g of succinic acid, 10 ml of propylene glycol, 10 ml of hydrogen peroxide, 5 g of sodium silicate, 0.1 g of stearic acid, 0.05 g of sodium dodecyl sulfonate, and 50 ml of water. A flow rate of the polishing solution was controlled to be 0.5 m/s, and the stent was polished at 30°C for 3 min. The stent was removed, cleaned, and dried. The dried stent was observed under an optical microscope with a magnification of 200 times to find that the surface was smooth and bright. The dried stent was observed under an SEM with a magnification of 500 times to find that edges of the stent were round, the percentage of a surface scratch area was 2%, the maximum depth of the scratch was 2 $\mu$m, the single-side removal depth of a stent rod was 15 $\mu$m, and the uniformity of stent polishing was 92%. A mass loss rate of the stent was 44%, and the roughness Sa of the surface of the stent measured under a sensofar 3D optical profiler was 20 nm. The stent had a radial strength of 120 kPa and was expanded using a balloon to a diameter of 4.6 mm without cracks and fracture. There was no obvious change after the stent was stored for 4 weeks. Five stents were consecutively and repeatedly polished, and the RSD of the mass of the polished stents was < 2.5%.

## Example 5

A 30018 stent was cut, ultrasonically cleaned for 2 min with 12.5% hydrochloric acid, and then placed in a solution with a viscosity of 1.0 mPa s, which contained 100 ml of hydrofluoric acid, 3 g of diethylene triamine pentamethylene phosphonic acid, 10 ml of cyclohexanol, 10 ml of potassium permanganate, 5 g of sodium alginate, 0.1 g of palmitic acid, and 50 ml of pure water. The solution was placed in a low-temperature magnetic groove at a temperature of 30°C. The polishing solution flowed at a rate of 0.8.0 m/s, and the stent was placed in the polishing solution for polishing for 3 min. The stent was removed, cleaned, and dried. The dried stent was observed under an optical microscope with a magnification of 200 times to find that the surface was smooth and bright. The dried stent was observed under an SEM with a magnification of 500 times to find that edges of the stent were round, the percentage of a surface scratch area was 2%, the maximum depth of the scratch was 0.5 $\mu$m, the single-side removal depth of a stent rod was 15 $\mu$m, and the uniformity of stent polishing was 92%. A mass loss rate of the stent was 46%, and the roughness Sa of the surface of the stent measured under a sensofar 3D optical profiler was 20 nm. The stent had a radial strength of 115 kPa and was expanded using a balloon to a diameter of 4.6 mm without cracks and fracture. Five stents were consecutively and repeatedly polished, and the RSD of the mass of the polished stents was < 3%.

## Example 6

A 30018 stent was cut, ultrasonically cleaned for 2 min with 12.5% hydrochloric acid, and then placed in a solution with a viscosity of 2.5 mPa s, which contained 100 ml of perchloric acid, 5 g of sodium gluconate, 10 ml of butanol, 5 g of potassium nitrate, 5 g of sodium alginate, 0.1 g of sodium stearate, 0.05 g of betaine, 0.05 g of sodium lauryl sulfate, and 50 ml of pure water. The solution was placed in a low-temperature magnetic groove at a temperature of 25°C. The polishing solution flowed at a rate of 0.6.0 m/s, and the stent was placed in the polishing solution for polishing for 3 min. The stent was removed, cleaned, and dried. The dried stent was observed under an optical microscope with a magnification of 200 times to find that the surface was smooth and bright. The dried stent was observed under an SEM with a magnification of 500 times to find that edges of the stent were round, the percentage of a surface scratch area was 2%, the maximum depth of the scratch was 0.3 $\mu$m, the single-side removal depth of a stent rod was 15 $\mu$m, and the uniformity of stent polishing was 95%. A mass loss rate of the stent was 43%, and the roughness Sa of the surface of the stent measured under a sensofar 3D optical profiler was 12 nm. The stent had a radial strength of 118 kPa and was expanded using a balloon to a diameter of 4.6 mm without cracks and fracture. Five stents were consecutively and repeatedly polished, and the RSD of the mass of the polished stents was < 3%.

## Example 7

A 30018 stent was cut, placed in an ultrasonic wave for cleaning for 2 min, and then placed in a polishing solution with a viscosity of 2.5 mPa s, which was prepared by mixing 100 ml of sulfuric acid, 5 g of disodium EDTA, 10 ml of ethylene glycol, 5 g of sodium nitrate, 0.1 ml of phytic acid, and 50 ml of water. A flow rate of the polishing solution was controlled to be 0.5 m/s, and the stent was polished at 25°C for 3 min. The stent was removed, cleaned, and dried. The dried stent was observed under an optical microscope with a magnification of 200 times to find that the surface was smooth and bright. The dried stent was observed under an SEM with a magnification of 500 times to find that edges of the stent were round, the percentage of a surface scratch area was 2.5%, the maximum depth of the scratch was 2 $\mu$m, the single-side removal depth of a stent rod was 15 $\mu$m, and the uniformity of stent polishing was 85%. A mass loss rate of the stent was 44%, and the roughness Sa of the surface of the stent measured under a sensofar 3D optical profiler was 18 nm. The stent had a radial strength of 115 kPa and was expanded using a balloon to a diameter of 4.5 mm with a fracture. Five stents were consecutively and repeatedly polished, and the RSD of the mass of the polished stents was < 3%.

## Example 8

A 30018 stent was cut, placed in an ultrasonic wave for cleaning for 2 min, and then placed in a polishing solution with a viscosity of 3.5 mPa s, which was prepared by mixing 100 ml of hydrochloric acid, 3 g of succinic acid, 10 ml of glycerol, 10 ml of nitric acid, 5 g of sodium alginate, and 50 ml of water. A flow rate of the polishing solution was controlled to be 0.6 m/s, and the stent was polished at 30°C for 3 min. The stent was removed, cleaned, and dried. The dried stent was observed under an optical microscope with a magnification of 200 times to find that the surface was smooth and bright. The dried stent was observed under an SEM with a magnification of 500 times to find that edges of the stent were round, the percentage of a surface scratch area was 2%, the maximum depth of the scratch was 1.2 $\mu$m, the single-side removal depth of a stent rod was 1.5 $\mu$m, and the uniformity of stent polishing was 88%. A mass loss rate of the stent was 48%, and the roughness Sa of the surface of the stent measured under a sensofar 3D optical profiler was 25 nm. The stent had a radial strength of 120 kPa and was expanded using a balloon to a diameter of 4.6 mm without cracks and fracture. The stent had yellow spots on the surface after 7 days of storage. Five stents were consecutively and repeatedly polished, and the RSD of the mass of the polished stents was < 2%.

## Example 9

A 30018 stent was cut, placed in an ultrasonic wave for cleaning for 2 min, and then placed in a polishing solution with a viscosity of 3.5 mPa s, which was prepared by mixing 100 ml of phosphoric acid, 5 g of malic acid, 10 ml of butanediol, 10 ml of perchloric acid, and 50 ml of water. A flow rate of the polishing solution was controlled to be 0.8 m/s, and the stent was polished at 35°C for 3 min. The stent was removed, cleaned, and dried. The dried stent was observed under an optical microscope with a magnification of 200 times to find that the surface was smooth and bright. The dried stent was observed under an SEM with a magnification of 500 times to find that edges of the stent were round, the percentage of a surface scratch area was 3%, the maximum depth of the scratch was 2.5 $\mu$m, the single-side removal depth of a stent rod was 15 $\mu$m, and the uniformity of stent polishing was 82%. A mass loss rate of the stent was 48%, and the roughness Sa of the surface of the stent measured under a sensofar 3D optical profiler was 30 nm. The stent had a radial strength of 128 kPa and was expanded using a balloon to a diameter of 4.0 mm with a fracture. Five stents were consecutively and repeatedly polished, and the RSD of the mass of the polished stents was < 1.5%.

## Comparative example 1

A 30018 stent was cut, placed in an ultrasonic wave for cleaning for 2 min, and then placed in a polishing solution, which was prepared by mixing 140 ml of glacial acetic acid and 60 ml of perchloric acid. The stent was polished using a direct current power supply at a constant current of 0.1 A for 20 s. The stent was removed, cleaned, and dried. The dried stent was observed under an optical microscope with a magnification of 200 times to find that the surface was smooth and bright. However, the stent was locally yellow and had small sizes at two ends and a large size in the middle. The dried stent was observed under an SEM with a magnification of 500 times to find that edges of the stent were round, the percentage of a surface scratch area was 5%, the maximum depth of the scratch was 6 μm, the single-side removal depth of an inner wall of a stent rod was 5 μm, and the uniformity of stent polishing was 50%. A mass loss rate of the stent was 50%, and the roughness Sa of the surface of the stent measured under a sensofar 3D optical profiler was 200 nm. The stent had a radial strength of 95 kPa and was expanded using a balloon to a diameter of 3.5 mm with a fracture. Five stents were consecutively and repeatedly polished, and the RSD of the mass of the polished stents was < 3%.

**Comparative example 2**

A 30018 stent was cut and placed in an ultrasonic wave for cleaning for 2 min. A polishing solution prepared by mixing 60 ml of phosphoric acid, 30 ml of sulfuric acid, and 10 ml of nitric acid was heated to boiling. The stent was placed in the heated polishing solution for polishing for 10 s, cleaned, and dried. The dried stent was observed under an optical microscope with a magnification of 200 times to find that the surface was rough, pitted, and yellow. The dried stent was observed under an SEM with a magnification of 500 times to find that edges of the stent were round, the percentage of a surface scratch area was 8%, the maximum depth of the scratch was 0.5 μm, the single-side removal depth of an inner wall of a stent rod was 16 μm, and the uniformity of stent polishing was 90%. A mass loss rate of the stent was 65%, and the roughness Sa of the surface of the stent measured under a sensofar 3D optical profiler was 250 nm. The stent had a radial strength of 70 kPa and was expanded using a balloon to a diameter of 4.0 mm with a fracture. Five stents were consecutively and repeatedly polished, and the RSD of the mass of the polished stents was > 10%.

It can be seen from the comparative examples that in comparative example 1, after electrochemical polishing, the sizes of various parts of the stent are uneven, the surface is yellow and relatively rough, the percentage of the surface scratch area is relatively high, the scratch has a large depth, and both the radial support force and the over-expansion capability of the stent are relatively low. In comparative example 2, after the high-temperature chemical polishing, the surface of the stent is yellow and rough, and the percentage of the surface scratch area of the polishing member is also relatively high. Meanwhile, the polishing time of this method is very short, only 5 s, and the polishing precision of the product is not high. After repeated polishing for many times, the RSD of product size and quality among multiple polished products is relatively large, and the polishing stability is poor.

The above are only preferred examples of the present invention and are not intended to limit the present invention. For example, the examples of the present invention are all illustrated by using a stent as an example, which does not mean that the solution of the present application is only suitable for the polishing of the stent.

The solution of the present application may also be suitable for the polishing of precision parts of other medical devices and even other non-medical devices with a small mass. Although the present invention has been described in detail with reference to the foregoing examples, a person skilled in the art will be able to make modifications to the technical solutions described in the foregoing examples or make equivalents to some of the technical features thereof. Any modifications, equivalents, improvements, etc. made within the spirit and principles of the present invention should be included within the scope of the present invention.

**Claims**

1. A polishing composition, comprising an acid, an oxidizing agent, and water; a pH value of the polishing composition being $\leq 1$,
   wherein the acid is selected from at least one of inorganic acids; the oxidizing agent is selected from at least one of strong inorganic oxidizing agents.

2. The polishing composition according to claim 1, wherein a mass percentage content of the inorganic acid is 40%-70%, and a mass percentage content of the strong inorganic oxidizing agent is 5%-10%.

3. The polishing composition according to claim 1, wherein the viscosity of a polishing solution is 1 mPa s-4 mPa s.

4. The polishing composition according to claim 1, wherein the inorganic acid is selected from at least one of sulfuric acid, phosphoric acid, nitric acid, perchloric acid, or hydrofluoric acid; the inorganic oxidizing agent is selected from at least one of sulfate, perchloric acid and salts thereof, perbromic acid and salts thereof, periodic acid and salts thereof, permanganic acid and salts thereof, peroxy acid salts, iodic acid and salts thereof, chlorous acid and salts thereof, hypochlorous acid and salts thereof, hypoiodous acid and salts thereof, oxybromic acid and salts thereof, percarbonate, bromic acid and salts thereof, chloric acid and salts thereof, hydrogen peroxide, potassium nitrate, or sodium nitrate.

5. The polishing composition according to claim 1, wherein the polishing composition further comprises a viscosity agent; a mass percentage content of the viscosity agent is 1.5-7%.

6. The polishing composition according to claim 5, wherein the viscosity agent is selected from at least one of alcohols, silicic acid, silicate, alginic acid, alginate, polyvinylpyrrolidone, polyvinylpyrrolidone-modified organic, ethyl cellulose, methyl cellulose, hydroxyethyl cellulose, hydroxypropylmethyl cellulose, carboxymethyl cellulose, polyacrylic acid, sodium polymethacrylate, polycarboxylic acid sodium salts, carboxylic acid sodium salt copolymers, carboxylic acid sulfonic acid copolymers, guar gum, sodium starch phosphate, sodium polyacrylate, polyoxyethylenes, carbomer, xanthic acid, arabic gum, carrageenan, agar, gelatin, acacia gum, and derivatives thereof.

7. The polishing composition according to claim 5, wherein the viscosity agent comprises at least one of A components and at least one of B components, wherein

the A component is selected from at least one of alcohols, ethyl cellulose, methyl cellulose, hydroxyethyl cellulose, hydroxypropylmethyl cellulose, carboxymethyl cellulose, polyvinylpyrrolidone, polyacrylic acid, sodium polymethacrylate, polycarboxylic acid sodium salts, carboxylic acid sodium salt copolymers, carboxylic acid sulfonic acid copolymers, and derivatives thereof;
the B component is selected from a mixture of at least one of silicic acid, silicate, alginic acid, alginate, polyvinylpyrrolidone-modified organic, guar gum, sodium starch phosphate, sodium polyacrylate, polyoxyethylenes, carbomer, xanthic acid, arabic gum, carrageenan, agar, gelatin, acacia gum, and derivatives thereof.

8. The polishing composition according to claim 7, wherein a mass ratio of the A component to the B component is 0.5: 1-15: 1.

9. The polishing composition according to claim 1, wherein the polishing composition further comprises a complexing agent, and the complexing agent is a monodentate or polydentate ligand; the complexing agent is selected from at least one of an amino group, a carboxyl group, a cyanide group, a thiocyanate group, an isothiocyanate group, a nitro group, a hydroxyl group, a mercapto group, an aromatic heterocyclic group, a nitroso group, a sulfo group, a phosphoric acid group, and an organic phosphine group.

10. The polishing composition according to claim 9, wherein a mass percentage content of the complexing agent is 0.5%-2%.

11. The polishing composition according to claim 1, wherein the polishing composition further comprises a surface protecting agent; a mass percentage content of the surface protecting agent is 0.02%-1%.

12. The polishing composition according to claim 11, wherein the surface protecting agent is selected from at least one of tannic acid, phytic acid, stearic acid, palmitic acid, and salts thereof.

13. The polishing composition according to claim 1, wherein the polishing composition further comprises a surfactant; a mass percentage content of the surfactant is 0.05%-1%.

14. The polishing composition according to claim 13, wherein the surfactant is at least one of an anionic surfactant, a cationic surfactant, a nonionic surfactant, or an amphoteric surfactant; the surfactant is selected from at least one of higher fatty acid salts, alkyl sulfonate, alkyl ether carboxylate surfactants, silicone surfactants, alkane sulfate, alkane sulfonate, substituted amine salts, betaine, lecithin, amino acid derivatives, alkylphenol polyoxyethylene ether, fatty alcohol polyoxyethylene ether sodium sulfate, isooctanol polyoxyethylene ether, alkyl glucoside, or polyoxyethylene ether.

15. An application of the polishing composition according to any one of claims 1-14 in polishing a medical device, wherein the medical device comprising an interventional device or an implant device in vivo.

16. The application of the polishing composition in polishing a medical device according to claim 15, wherein the medical device is made of a degradable metal or a metal alloy; the medical device comprises a vascular stent, a non-vascular endoluminal stent, an occluder, a spacer, an artificial vessel, a dental implant device, a vascular clamp, an artificial heart valve, an orthopedic implant, a dental implant, a respiratory implant, a gynecological implant, an andrological implant, a suture, or a bolt.

**FIG. 1**

**FIG. 2**

**FIG. 3**

## INTERNATIONAL SEARCH REPORT

| International application No. |
|---|
| **PCT/CN2023/110247** |

| A. | CLASSIFICATION OF SUBJECT MATTER |
|---|---|

C23F3/00(2006.01)i

According to International Patent Classification (IPC) or to both national classification and IPC

| B. | FIELDS SEARCHED |
|---|---|

Minimum documentation searched (classification system followed by classification symbols)

C23F

Documentation searched other than minimum documentation to the extent that such documents are included in the fields searched

Electronic data base consulted during the international search (name of data base and, where practicable, search terms used)

CNTXT, ENTXTC, DWPI, 中国期刊网全文数据库, Chinese Journal Full-text Database: 元心科技; 医; 药; 支架; 植入; 血管; 酸; 过硫酸; 高氯酸; 高溴酸; 高碘酸; 高锰酸; 过氧酸; 碘酸; 亚氯酸; 次氯酸; 次碘酸; 氧溴酸; 过碳酸; 溴酸; 氯酸; 双氧水; 过氧化氢; 硝酸; fe; 铁; mg; 镁; zn; 锌; 粘稠; 粘度; 抛光; polish+ ; implant+ ; medic+; stent? ; remov+; acid

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 101851470 A (BYD CO., LTD.) 06 October 2010 (2010-10-06)<br>claims 1-10 | 1, 4 |
| Y | CN 108193207 A (ZHANG HAIJUN) 22 June 2018 (2018-06-22)<br>description, paragraphs 6 and 8-12, and claim 8 | 1-16 |
| Y | CN 111472004 A (SOUTHEAST UNIVERSITY) 31 July 2020 (2020-07-31)<br>description, paragraphs 5-18 | 1-16 |
| A | CN 101297777 A (JIANGYIN FASTEN-PLT MATERIALS SCIENCE CO., LTD.) 05 November 2008 (2008-11-05)<br>entire document | 1-16 |
| A | CN 106086908 A (DONGGUAN KAIMENG SURFACE TREATMENT TECHNOLOGY DEVELOPMENT CO., LTD.) 09 November 2016 (2016-11-09)<br>entire document | 1-16 |
| A | US 4704126 A (RICHARDS MEDICAL COMPANY) 03 November 1987 (1987-11-03)<br>entire document | 1-16 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **07 November 2023** | **10 November 2023** |

| Name and mailing address of the ISA/CN | Authorized officer |
|---|---|
| **China National Intellectual Property Administration (ISA/ CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2023/110247**

| Patent document cited in search report | | | Publication date (day/month/year) | Patent family member(s) | | | Publication date (day/month/year) |
|---|---|---|---|---|---|---|---|
| CN | 101851470 | A | 06 October 2010 | CN | 101851470 | B | 13 March 2013 |
| CN | 108193207 | A | 22 June 2018 | None | | | |
| CN | 111472004 | A | 31 July 2020 | None | | | |
| CN | 101297777 | A | 05 November 2008 | CN | 101297777 | B | 09 June 2010 |
| CN | 106086908 | A | 09 November 2016 | CN | 106086908 | B | 24 August 2018 |
| US | 4704126 | A | 03 November 1987 | JPS | 6264366 | A | 23 March 1987 |
| | | | | JPH | 0236266 | B2 | 16 August 1990 |
| | | | | EP | 0202031 | A2 | 20 November 1986 |
| | | | | EP | 0202031 | B1 | 15 January 1992 |
| | | | | DE | 3683395 | D1 | 27 February 1992 |
| | | | | CA | 1252697 | A | 18 April 1989 |

Form PCT/ISA/210 (patent family annex) (July 2022)

**EP 4 563 724 A1**

**Patent documents cited in the description**

- CN 102356184 B